# EUROPÄISCHE PATENTSCHRIFT

(11) **EP 4 631 587 B1**
(45) Veröffentlichungstag und Bekanntmachung des Hinweises auf die Patenterteilung: **14.10.2026**
(21) Anmeldenummer: 24169038.7
(22) Anmeldetag: 08.04.2024
(51) Int. Cl.: A63B 71/14, A63B 71/08, A63B 24/00, G01L 1/00, G01L 5/00, G06F 1/00, A61B 5/00, G06F 1/16, G06V 40/20, G09B 19/00, A63B 71/06, G06F 3/01

(54) **MODULARES SCHLAGEQUIPMENT**
MODULAR STRIKING EQUIPMENT
DISPOSITIF MODULAIRE DE FRAPPE

(43) Veröffentlichungstag der Anmeldung: 15.10.2025
(73) Patentinhaber: Research Industrial Systems Engineering (RISE) Forschungs-, Entwicklungs- und Grossprojektberatung GmbH, 2320 Schwechat (AT)
(72) Erfinder: HÖLBLING, Dominik, 2482 Münchendorf (AT); GRECHENIK, Thomas, 1040 Wien (AT)
(74) Vertreter: Schwarz & Partner Patentanwälte GmbH

(56) Entgegenhaltungen:
- KR-A- 20130 085 868
- RU-U1- 207 390
- US-A1- 2011 251 802
- US-A1- 2012 144 554
- US-A1- 2012 266 358
- US-A1- 2015 375 042

## Beschreibung

Die Erfindung betrifft ein modulares Schlagequipment, bevorzugt einen modularen Schlaghandschuh, zur Bestimmung einer Schlageigenschaft, insbesondere einer Schlagbeschleunigung, Schlaggeschwindigkeit, Schlagkraft oder Schlagtechnik, umfassend einen Schlagkörper und ein Elektronikmodul, wobei der Schlagkörper einen Dämpfungskörper und einen Drucksensor umfasst, wobei das Elektronikmodul zumindest eine elektronische Komponente, bevorzugt eine erste Recheneinheit zur Bestimmung der Schlageigenschaft, umfasst.

Bei Kampfsportarten treten üblicherweise zwei oder mehr Athleten in einem Ring gegeneinander an und versuchen, mittels Schlägen, Tritten oder sonstigen Körperkontakten Treffer am jeweils anderen Athleten zu landen. Beispiele für derartige Kampfsportarten, die Gegenstand dieser Beschreibung sind, sind Boxen, Karate, Kickboxen, Taekwondo, Kung Fu etc.

Zu Wettkampfzwecken aber auch für Trainings und sonstige Tests ist es wünschenswert, einen Schlag oder Tritt oder deren Wirkung auf ein Körperteil zu klassifizieren, z.B. indem man dem Schlag oder Tritt eine Schlagfrequenz, Beschleunigung, Kraft, einen von der Beschleunigung oder der Kraft abgeleiteten Wert, oder eine daraus kombinierte bzw. aus einer Analyse abgeleitete Variable, wie die Schlagtechnik zuordnet. Zur Messung der Beschleunigung sind verschiedene Varianten bekannt, z.B. mittels einer Videoauswertung der Bewegung der Athleten oder mittels einer in einem Schlaghandschuh verbauten Sensorik, die Beschleunigungen und/oder Rotationsgeschwindigkeiten erfassen (Inertial Measurement Unit, IMU). Stellvertretend werden hierfür die Schriften US 2017/134712, die US 2018/001141, die US 2012/144414 und die WO 2019/106672 genannt.

Im Stand der Technik ist ein Schlaghandschuh beispielsweise aus der EP 3844468 bekannt. Der offenbarte Schlaghandschuh umfasst einen Schlagkörper mit einem Dämpfungskörper, einem fluidgefüllten Körper und einem Drucksensor zur Messung des hydrostatischen Drucks im fluidgefüllten Körper. Bei diesem Schlaghandschuh sind neben dem Drucksensor auch die Recheneinheit, ein Beschleunigungssensor, eine Batterie und ein Sendeempfänger innerhalb des Schlagkörpers angeordnet. Alle Komponenten sind im Schlaghandschuh eingenäht, um einen kompakten Schlaghandschuh auszubilden.

Nachteilig ist bei dem genannten Schlaghandschuh, dass ein Defekt einer dieser Komponenten den gesamten Schlaghandschuh unbenützbar macht. Ebenso führt ein Defekt im fluidgefüllten Körper unmittelbar dazu, dass der Schlaghandschuh als Ganzes nicht mehr benützbar ist. Weiters muss zum Aufladen der Batterie der Schlaghandschuh zu einem entsprechenden Ladegerät verbracht werden, was die Handhabung des Schlaghandschuhs verkompliziert, da sichergestellt werden muss, dass der Schlaghandschuh vor einem Wettkampftag oder Trainingstag vollständig aufgeladen ist. Würde die Batterie dennoch im Laufe eines Wettkampftages leer, muss man zum Beispiel zumindest eine Stunde Wartezeit in Kauf nehmen, bis die Batterie wieder ausreichend aufgeladen ist. Ein weiterer Nachteil besteht darin, dass beim Transport in einem Flugzeug der ganze Schlaghandschuh im Handgepäck mitgeführt werden muss.

Weiters ist von der Firma ROOQ^{®} ein Sensor bekannt, der über Stoffbänder am Unterarm eines Athleten befestigt werden kann und mittels Beschleunigungssensoren Schlagkraft, Schnelligkeit und Schlaganzahl erfassen kann. Diese Daten werden im Sensor gespeichert und können nach dem Training in einer Smartphone-App ausgewertet werden. Nachteilig an diesem Sensor ist, dass die Schlagkraft nicht exakt bestimmt werden kann, da zur Bestimmung der Schlagkraft ausschließlich mit Messdaten eines Beschleunigungssensors eine geschätzte effektive Masse herangezogen werden muss.

Aus der KR 2013 0085868 A ist ein elektronisches Schlagequipment bekannt, bei dem ein Schlagmodul mit einem Drucksensor und einem Beschleunigungssensor über ein Kabel mit einem Controller verbunden ist. Aus der US 2015/0375042 A1 ist ferner ein modulares Aktivitätsüberwachungssystem bekannt, bei dem ein entnehmbares Elektronikmodul ("puck") mit einer Inertialmesseinheit in verschiedenen tragbaren Artikeln aufgenommen werden kann.

Es ist die Aufgabe der vorliegenden Erfindung, ein Schlagequipment zur Verfügung zu stellen, das zumindest einzelne Nachteile des Standes der Technik überwindet.

Diese Aufgabe wird durch ein modulares Schlagequipment, bevorzugt durch einen modularen Schlaghandschuh, zur Bestimmung einer Schlageigenschaft, insbesondere einer Schlagbeschleunigung, Schlaggeschwindigkeit, Schlagkraft oder Schlagtechnik, gelöst, das einen Schlagkörper und ein Elektronikmodul umfasst, wobei der Schlagkörper einen Dämpfungskörper und einen Drucksensor umfasst, wobei das Elektronikmodul zumindest eine elektronische Komponente, bevorzugt eine erste Recheneinheit zur Bestimmung der Schlageigenschaft umfasst, wobei das Elektronikmodul und der Schlagkörper jeweils eine Schnittstelle aufweisen, über welche der Drucksensor oder eine mit dem Drucksensor verbundene, im Schlagkörper vorliegende zweite Recheneinheit mit der zumindest einen elektronischen Komponente des Elektronikmoduls verbindbar ist, wobei das Elektronikmodul und der Schlagkörper physisch an den genannten Schnittstellen voneinander trennbar und zusammensetzbar sind, sodass das Elektronikmodul und der Schlagkörper in einen zusammengesetzten Zustand und in einen getrennten Zustand bringbar sind, wobei das modulare Schlagequipment in einem zusammengesetzten Zustand dazu ausgebildet ist, Daten und/oder Energie zwischen der zumindest einen elektronischen Komponente und dem Drucksensor und/oder der zweiten Recheneinheit über die genannten Schnittstellen auszutauschen. Bevorzugte Ausführungsformen sind in den abhängigen Ansprüchen, der Beschreibung und den Zeichnungen angegeben.

Ein besonderer Vorteil des erfindungsgemäßen Schlagequipments liegt darin, dass der Schlagkörper an sich auch ohne Recheneinheit (und gegebenenfalls auch ohne Beschleunigungssensor und Batterie) hergestellt werden kann, da der Schlagkörper erfindungsgemäß unabhängig vom Elektronikmodul hergestellt werden kann und eine Verbindung der beiden Elemente erst kurz vor dem bestimmungsgemäßen Gebrauch erfolgen kann. In der Praxis ist dies deshalb besonders vorteilhaft, da der Schlagkörper vorrangig aus textilen Materialien besteht, die in einer ersten Fertigungsstätte zum Schlagkörper zusammengefügt werden. Das Elektronikmodul würde in diesem Fall den Großteil der elektronischen Komponenten umfassen, sodass dieses in einer zweiten Fertigungsstätte zusammengefügt werden kann. Es ist daher nicht notwendig, alle elektronischen Komponenten in einem in Wesentlichen zusammengesetzten Zustand zur ersten Fertigungsstätte zu transportieren, wo diese in das Schlagequipment, beispielsweise einen Schlaghandschuh, eingenäht werden müssten, wie dies bei vergleichbaren Schlaghandschuhen aus dem Stand der Technik der Fall war. In Summe ergibt sich ein enormer logistischer Vorteil, da die beiden Fertigungsstätten üblicherweise in unterschiedlichen Ländern oder sogar in unterschiedlichen Kontinenten angesiedelt sind. Zusammengefasst kann insbesondere der notwendige Transportweg für die elektronischen Komponenten und insbesondere die Batterie, die ein Gefahrengut darstellt, reduziert werden.

Ein weiterer besonderer Vorteil ist, dass das Schlagequipment nicht allein deswegen defekt ist, weil etwa der Dämpfungskörper, der Drucksensor oder eine elektronische Komponente defekt ist. Das Schlagequipment steht unter besonderer Belastung, da bei jedem Schlag, Tritt oder dergleichen eine Kraft auf den Dämpfungskörper, den Drucksensor oder auch auf die elektronischen Komponenten ausgeübt wird. Durch die modulare Ausführung kann in im Falle eines Defekts einfach ein anderer Schlagkörper mit dem weiterhin funktionstüchtigen Elektronikmodul oder ein anderes Elektronikmodul mit dem weiterhin funktionstüchtigen Schlagkörper verbunden werden.

Die genannten Vorteile werden erfindungsgemäß durch die Einführung der Schnittstellen erzielt, über welche der Schlagkörper und das Elektronikmodul verbindbar und trennbar sind, wobei eine Kommunikation zwischen dem Schlagkörper und dem Elektronikmodul durchführbar ist. Unter "verbindbar und trennbar" wird hierin verstanden, dass ein Benutzer den Schlagkörper und das Elektronikmodul unkompliziert, vorzugsweise ohne Hilfsmittel wie Werkzeuge, verbinden und auch wieder trennen kann.

Im Rahmen der vorliegenden Erfindung kann unter einer Schnittstelle beispielsweise eine physische Schnittstelle wie eine Hardwareschnittstelle oder eine Netzwerkschnittstelle verstanden werden. Eine solche erfindungsgemäße Schnittstelle kann beispielsweise durch elektrische Kontakte oder durch eine Steckverbindung, etwa eine USB-Verbindung, realisiert werden. Falls die Schnittstelle eine drahtlose Kommunikation ermöglicht, kann die Schnittstelle durch geeignete Sende- und Empfangseinheiten für WLAN, Bluetooth, NFC oder dergleichen realisiert werden. Insbesondere wird unter Schnittstelle keine reine softwarebasierte Schnittstelle, etwa eine Softwareschnittstelle, oder eine Schnittstelle zwischen Mensch und Maschine, etwa eine Benutzerschnittstelle, verstanden. Da die Schnittstellen lösbar sein sollen, wird unter einer Schnittstelle auch keine Lötverbindung oder dergleichen verstanden.

Es ist weiters vorteilhaft, wenn das modulare Schlagequipment gegen eine Kraftmesseinheit, beispielsweise eine Kraftmessplatte, validiert wird, um eine akkurate Schlagkraft ermitteln zu können. Konkret kann bei einem Schlag mit dem Schlagequipment auf eine Kraftmessplatte der vom Drucksensor gemessene Druck und die von der Kraftmessplatte gemessene Kraft gemessen werden, sodass Druckwerten des Drucksensors eine Kraft zugeordnet werden kann.

An dieser Stelle sei angemerkt, dass der Drucksensor in allen Ausführungsformen als piezoresistiver Drucksensor (auch DMS-Drucksensor genannt), piezoelektrischer Drucksensor (beispielsweise eine piezoelektrische Sensormatte) und/oder als kapazitiver Drucksensor ausgebildet sein kann.

Erfindungsgemäß umfasst das Elektronikmodul die erste Recheneinheit und einen Beschleunigungssensor als elektronische Komponente, wobei die erste Recheneinheit dazu ausgebildet ist, in einem getrennten Zustand eine Schlageigenschaft über vom Beschleunigungssensor erhaltenen Beschleunigungsmessdaten zu ermitteln und/oder die Beschleunigungsmessdaten zur Ermittlung einer Schlageigenschaft an eine externe Recheneinheit zu senden. Erfindungsgemäß kann das Elektronikmodul somit auch im getrennten Zustand, also selbstständig und unabhängig vom Schlagkörper eine Schlageigenschaft ermitteln. Im zusammengesetzten Zustand ist eine genauere Bestimmung einer Schlagkraft über die Druckmessdaten des Drucksensors im Schlagkörper möglich.

In einer bevorzugten Ausführungsform umfasst der Schlagkörper einen fluidgefüllten Körper, in welchem der Drucksensor angeordnet ist, um einen hydrostatischen Druck im fluidgefüllten Körper zu messen. Dies ermöglicht die Verwendung eines besonders einfach gestalteten Drucksensors, da sich bei Kraftausübung auf einen beliebigen Punkt des fluidgefüllten Körpers der hydrostatische Druck in seinem Inneren überall gleichmäßig erhöht. Der Drucksensor muss also nicht die komplette Fläche abdecken, entlang welcher eine Kraftausübung zu erwarten ist.

In einer bevorzugten Ausführungsform umfasst das Elektronikmodul die erste Recheneinheit als elektronische Komponente, welche dazu ausgebildet ist, eine Schlageigenschaft über vom Drucksensor erhaltenen Druckmessdaten zu ermitteln und/oder die vom Drucksensor erhaltenen Druckmessdaten zur Ermittlung einer Schlageigenschaft an eine externe Recheneinheit zu senden, wenn sich das Elektronikmodul und der Schlagkörper im zusammengesetzten Zustand befinden. Falls das Elektronikmodul die vom Drucksensor erhaltenen Druckmessdaten zur Ermittlung einer Schlageigenschaft an eine externe Recheneinheit, beispielsweise ein Smartphone, sendet, kann die Auswertung der Messdaten am Smartphone erfolgen, sodass die erste Recheneinheit besonders einfach ausgestaltet sein kann. In anderen Worten muss in diesem Fall die erste Recheneinheit keine große Rechenleistung aufweisen können.

In einer bevorzugten Ausführungsform umfasst der Schlagkörper die zweite Recheneinheit zur Bestimmung der Schlageigenschaft auf Basis der vom Drucksensor gelieferten Messdaten und/oder einen Sendeempfänger. In dieser Ausführungsform kann der Schlagkörper selbstständig eine Schlageigenschaft ermitteln. Das Elektronikmodul kann hierbei auch die erste Recheneinheit umfassen, welche hierbei bloß als Sendeempfänger dienen kann oder auch die Schlageigenschaft redundant ermitteln kann, sodass selbst bei einem teilweisen Defekt einer der beiden Recheneinheiten die Schlageigenschaft bestimmt oder eine Übertragung der Messdaten an eine externe Recheneinheit erfolgen kann. Zusammengefasst können die elektronischen Komponenten sowohl im Schlagkörper als auch im Elektronikmodul vorgesehen und damit verdoppelt werden, um eine Redundanz zu erzeugen. In einer besonders einfachen Ausführungsform kann das Elektronikmodul jedoch auch lediglich eine Batterie als elektronische Komponente umfassen, die die zweite Recheneinheit, den Beschleunigungssensor und/oder den Sendeempfänger mit Energie versorgt.

Besonders bevorzugt ist jedoch, wenn das Elektronikmodul selbst den Beschleunigungssensor umfasst und dazu ausgebildet ist, in einem getrennten Zustand eine Schlageigenschaft über vom Beschleunigungssensor erhaltenen Beschleunigungsmessdaten in einer ersten Recheneinheit zu ermitteln. Dies hat den Vorteil, dass das Elektronikmodul dazu eingesetzt werden kann, eine Schlageigenschaft wie die Schlagkraft zu ermitteln, auch wenn der Drucksensor nicht mit dem Elektronikmodul verbunden ist. In andern Worten kann das Elektronikmodul somit in zwei Betriebsarten verwendet werden. Erstens kann der Benutzer das Elektronikmodul mit dem Schlagkörper verbinden, wodurch dieses die Schlageigenschaft besonders genau über die Druckmesswerte ermitteln kann. Zweitens kann der Benutzer das Elektronikmodul jedoch auch eigenständig als "Stand-Alone"-Produkt verwenden, bei dem es nicht mit dem Schlagkörper bzw. dem Drucksensor verbunden ist, und dennoch eine Schlageigenschaft über die vom Beschleunigungssensor erhaltenen Beschleunigungsmessdaten ermitteln kann, auch wenn dies ungenauer ist als über die Druckmesswerte.

Das Elektronikmodul kann bevorzugt dazu ausgebildet sein, im getrennten Zustand eine Schlagkraft auf Basis der vom Beschleunigungssensor erhaltenen Beschleunigungsmessdaten und einer geschätzten effektiven Masse zu ermitteln. Bei Kenntnis einer effektiven Masse liegt der Vorteil dieser Ausführungsform darin, dass selbst ohne Kommunikation mit einem Drucksensor eine Schlagkraft ermittelt werden kann. Die effektive Masse könnte hierfür als konstanter Wert in der Recheneinheit hinterlegt sein oder über eine bzw. die Schnittstelle an die Recheneinheit kommuniziert werden. Alternativ kann die effektive Masse in einer externen Datenverarbeitungseinheit wie einem Smartphone hinterlegt sein. Besonders bevorzugt kann das Elektronikmodul im zusammengesetzten Zustand einen geschätzten Wert für die effektive Masse ermitteln und diesen Wert zu einem späteren Zeitpunkt im getrennten Zustand zur Ermittlung einer Schlagkraft auf Basis der vom Beschleunigungssensor erhaltenen Beschleunigungsmessdaten heranzuziehen.

In einer Ausführungsform ist das Elektronikmodul dazu ausgebildet, im zusammengesetzten Zustand eine Schlagkraft auf Basis der vom Drucksensor erhaltenen Druckmessdaten und einer Querschnittsfläche des fluidgefüllten Körpers zu ermitteln. Der Vorteil dieser Ausführungsform liegt darin, dass eine Bestimmung einer Schlagkraft über den Drucksensor einen genaueren Wert liefert, im Vergleich dazu, dass die Schlagkraft über eine geschätzte effektive Masse ermittelt wird. Die Querschnittsfläche des fluidgefüllten Körpers könnte hierfür in der Recheneinheit hinterlegt sein. Alternativ oder zusätzlich könnte der Wert der Querschnittsfläche des fluidgefüllten Körpers über die Schnittstellen vom Schlagkörper an das Elektronikmodul übertragen werden. Weiters alternativ oder zusätzlich kann der Wert der Querschnittsfläche des fluidgefüllten Körpers in einer externen Datenverarbeitungseinheit wie einem Smartphone hinterlegt sein.

In einer Ausführungsform weist das Elektronikmodul einen Sendeempfänger als elektronische Komponente auf, der dazu ausgebildet ist, Daten vorzugsweise drahtlos und/oder kabelgebunden, an eine externe Datenverarbeitungseinheit zu übertragen. Die externe Datenverarbeitungseinheit könnte beispielsweise ein Mobiltelefon sein, sodass die ermittelte Schlageigenschaft über eine Applikation des Mobiltelefons live und/oder im Nachhinein als zusammenfassende Analyse ausgebbar ist.

In einer bevorzugten Ausführungsform umfasst das Elektronikmodul einen Signalindikator, bevorzugt eine LED-Anzeige und/oder ein LCD-Display, zum Mitteilen eines Betriebszustandes des Elektronikmoduls. Der Signalindikator könnte einem Benutzer unterschiedlichste Betriebszustände mitteilen. Beispielsweise könnte der Signalindikator ein Kopplungs- oder Entkopplungsvorgang des Elektronikmoduls zu bzw. von einem Schlagkörper mitteilen. Optional kann ein aufgetretener Fehler beim Kopplungsvorgang über den Signalindikator mitgeteilt werden. Alternativ oder zusätzlich kann das Bestehen oder das Nichtbestehen einer Verbindung über den Signalindikator einem Benutzer mitgeteilt werden. In einem praktischen Beispiel kann eine LED-Anzeige ein Licht anzeigen, insbesondere ein grünes Licht, wenn sich der Schlagkörper und das Elektronikmodul in einem zusammengesetzten Zustand befinden. Bei einem Verbindungsaufbau könnte die LED-Anzeige blinken. Es ist jedoch auch denkbar, dass es sich bei dem Signalindikator um einen akustischen, haptischen und/oder olfaktorischen Signalindikator handelt. Auch eine Kombination der genannten Signalindikatoren ist denkbar.

In einer bevorzugten Ausführungsform kann der Signalindikator einem Benutzer einen Verbindungszustand zum Drucksensor und/oder zur zweiten Recheneinheit, vorzugsweise das Herstellen einer Verbindung, das Bestehen einer Verbindung und/oder das Nichtbestehen einer Verbindung, mitteilen. Dies ermöglicht das schnelle Erkennen durch einen Benutzer, ob das modulare Schlagequipment im zusammengesetzten Zustand einsatzbereit ist. Alternativ oder zusätzlich kann der Signalindikator auf einer externen Datenverarbeitungseinheit, beispielsweise einem Smartphone, realisiert sein.

In einer bevorzugten Ausführungsform sind die Schnittstellen durch drahtlose Kommunikationsmittel gebildet. Ein Vorteil dieser Ausführungsform liegt darin, dass keine elektrischen Kontakte freiliegen und somit auch nicht anfällig für Verschleiß sind. Beispielsweise könnte Regenwasser und/oder Schweiß einen elektrischen Kontakt beschädigen. Außerdem kann es beim Aufprall zu einem kurzzeitigen Lösen der elektrischen Kontakte kommen (Wackelkontakt), sodass zu diesem Zeitpunkt die gewünschten Messgrößen nicht gemessen werden können. Wenn die Schnittstellen durch drahtlose Kommunikationsmittel gebildet sind, entfällt dieses Risiko. Besonders bevorzugt liegt hierbei innerhalb des Elektronikmoduls auch eine induktiv ladbare Batterie vor, sodass das Elektronikmodul überhaupt keine elektrischen Kontakte nach außen aufweisen muss.

Es sei noch erwähnt, dass, wenn die Schnittstellen durch drahtlose Kommunikationsmittel gebildet sind, es empfehlenswert ist, wenn der Schlagkörper neben dem Drucksensor auch einen Sendeempfänger und ein Datenverarbeitungselement umfasst. Die drahtlose Kommunikation könnte neben den Daten auch Energie übertragen, wie es etwa bei Nahfeldkommunikation (NFC) der Fall ist. Die drahtlose Kommunikation benötigt mehr Energie durch Erzeugen eines elektromagnetischen Feldes. Somit ist es bevorzugt, dass bei einer drahtlosen Schnittstelle diese nur während einer (potentiellen) Schlagbewegung Energie überträgt, sonst aber die meiste Zeit keine Kommunikation durchführt ist. Alternativ oder zusätzlich könnte zyklisch, beispielsweise alle x Sekunden getestet werden, ob eine Verbindung zum Drucksensor besteht.

In einer bevorzugten Ausführungsform umfasst das Elektronikmodul eine Batterie als elektrische Komponente, wobei die Batterie im getrennten Zustand über die genannte Schnittstelle des Elektronikmoduls aufladbar ist. Das ermöglicht eine platzsparende Konstruktion, sodass nur eine Schnittstelle im Elektronikmodul angeordnet sein kann. Im zusammengesetzten Zustand können hierbei die Druckmessdaten über die Schnittstelle an die Recheneinheit geliefert werden und im getrennten Zustand kann die Batterie über dieselbe Schnittstelle aufgeladen werden. In einer praktischen Ausführungsform könnte die Schnittstelle des Elektronikmoduls ein Anschluss wie ein USB-Anschluss sein, der sowohl eine Datenübertragung als auch ein Aufladen einer Batterie ermöglicht.

In einer bevorzugten Ausführungsform umfasst das Elektronikmodul weiters ein Gehäuse, welches alle elektronischen Komponenten des Elektronikmoduls, bevorzugt wasserdicht und/oder staubdicht, umschließt. Durch das Gehäuse kann das Elektronikmodul als abgekapselte Einheit bereitgestellt werden, und auch die Stabilität des Elektronikmoduls kann durch das Gehäuse erhöht werden. Insbesondere bei Kampfsportarten wie Boxen, Karate, Kickboxen, Taekwondo, Kung Fu etc. könnte das Elektronikmodul mit Schweiß in Berührung kommen. In diesen Fällen wäre das Elektronikmodul durch ein wasserdichtes Gehäuse vor flüssigkeitsbedingten Schäden geschützt. Vorzugsweise ermöglicht das Gehäuse einen Schutz vor Wasser und/oder Staub gemäß dem IP-Code IP11, gemäß dem IP-Code IP22 oder gemäß dem IP-Code IP33. Die erwähnten IP-Codes beziehen sich hierbei auf die Norm DIN EN 60529 in der zum Anmeldezeitpunkt letztgültigen Fassung.

In einer bevorzugten Ausführungsform sind alle elektrischen Kontakte, insbesondere Ladekontakte, des Elektronikmoduls im zusammengesetzten Zustand an der Schnittstelle des Elektronikmoduls abgedeckt. So kann ein Verschleiß durch Umwelteinflüsse vermieden werden. In anderen Worten liegt kein weiterer elektrischer Kontakt (z.B. zum Laden einer Batterie) an einer der vorgenannten Schnittstelle des Elektronikmoduls gegenüberliegenden oder angrenzenden Seite vor, der im zusammengesetzten Zustand frei liegt und dadurch ein Eindringen von Schweiß oder anderen Flüssigkeiten in das Elektronikmodul ermöglichen würde.

In einer bevorzugten Ausführungsform umfasst der Schlaghandschuhkörper weiters eine Aufnahmeeinheit, in welche das Elektronikmodul einführbar ist, wobei im zusammengesetzten Zustand das Elektronikmodul in der Aufnahmeeinheit zumindest teilweise, bevorzugt vollständig, eingeführt ist. Durch das Einführen des Elektronikmoduls in die Aufnahmeeinheit kann das physische Zusammenbringen der Schnittstellen erfolgen, sodass der zusammengesetzte Zustand hergestellt wird. Die Aufnahmeeinheit verhindert dabei insbesondere ein Lösen der beiden Schnittstellen und schafft damit eine erhöhte Betriebssicherheit. Falls die Schnittstellen als elektrische Kontakte ausgebildet sind, kann die Aufnahmeeinheit in ihrem Inneren ebenfalls elektrische Kontakte aufweisen. Falls die Schnittstellen eine drahtlose Kommunikation ermöglichen, könnte die Schnittstelle der Aufnahmeeinheit entsprechende Kommunikationsmittel aufweisen.

In einer bevorzugten Ausführungsform umfasst die Aufnahmeeinheit Dichtungselemente, welche die Schnittstellen im zusammengesetzten Zustand wasserdicht und/oder staubdicht im Innenraum der Aufnahmeeinheit einschließen. Falls die Schnittstellen als elektrische Kontakte ausgebildet sind, sind sie dadurch von Flüssigkeiten, beispielsweise Regenwasser oder Schweiß, geschützt. Vorzugsweise ermöglichen die Dichtungselemente einen Schutz vor Wasser und/oder Staub gemäß dem IP-Code IP11, gemäß dem IP-Code IP22 oder gemäß dem IP-Code IP33. Die erwähnten IP-Codes beziehen sich auf die Norm DIN EN 60529 in der zum Anmeldezeitpunkt letztgültigen Fassung.

In einem weiteren Aspekt der Erfindung wird ein System aus einem modularen Schlagequipment und einer Ladeeinheit bereitgestellt, wobei das Elektronikmodul im getrennten Zustand mit der Ladeeinheit verbindbar ist, wobei die Ladeeinheit dazu konfiguriert ist, eine Batterie des Elektronikmoduls über die Schnittstelle des Elektronikmoduls aufzuladen. Das ermöglicht ein unkompliziertes Aufladen der Batterie, da das Elektronikmodul in der Regel kleiner ist als der Schlagkörper und somit ohne großen Platzaufwand über die Ladeeinheit aufgeladen werden kann. Ein weiterer Vorteil liegt darin, dass der vom Schweiß des Athleten befeuchtete Schlagkörper während des Aufladens der Batterie des Elektronikmoduls, gegebenenfalls an einem anderen Ort, trocknen kann.

Das Elektronikmodul des modularen Schlagequipments kann eine Recheneinheit zur Bestimmung der Schlageigenschaft umfassen, wobei die Recheneinheit bevorzugt dazu ausgebildet ist, in einem ersten Zustand eine Schlageigenschaft über von einem Beschleunigungssensor erhaltenen Beschleunigungsmessdaten zu ermitteln. Das Elektronikmodul weist eine Schnittstelle auf, über welche die Recheneinheit mit einem Drucksensor verbindbar ist, wobei das Elektronikmodul in einem mit dem Drucksensor verbundenen zweiten Zustand dazu ausgebildet sein kann, eine Schlageigenschaft über vom Drucksensor erhaltenen Druckmessdaten zu ermitteln. Dadurch kann ein Elektronikmodul bereitgestellt werden, das modular mit einem Schlagkörper verbindbar ist. Dieses Elektronikmodul weist im Wesentlichen dieselben Vorteile auf und kann mit denselben vorteilhaften Ausführungsformen versehen werden, die oben für das Schlagequipment erläutert wurden. Besonders bevorzugt ist, wenn das Elektronikmodul einen Beschleunigungssensor und eine Batterie umfasst, wobei die Recheneinheit dazu ausgebildet ist, in einem ersten Zustand, in dem das Elektronikmodul bevorzugt nicht mit dem Drucksensor verbunden ist, eine Schlageigenschaft über vom Beschleunigungssensor erhaltenen Beschleunigungsmessdaten zu ermitteln. Dadurch kann das Elektronikmodul auch verwendet werden, wenn dieses nicht mit dem Drucksensor verbunden ist.

Vorteilhafte und nicht einschränkende Ausführungsformen der in den Ansprüchen wiedergegebenen Erfindung werden nachfolgend anhand der Zeichnungen näher erläutert.
Fig. 1 zeigt einen bekannten Schlaghandschuh aus dem Stand der Technik.
Fig. 2 zeigt eine Ausführungsform des erfindungsgemäßen modularen Schlagequipments als modularer Schlaghandschuh.
Fig. 3 zeigt eine vergrößerte Ansicht eines Elektronikmoduls.
Fig. 4 zeigt eine weitere Ausführungsform des erfindungsgemäßen modularen Schlagequipments als modularer Schlaghandschuh mit einer Ladeeinheit.
Fig. 5 zeigt eine weitere Verwendung eines erfindungsgemäßen Elektronikmoduls.

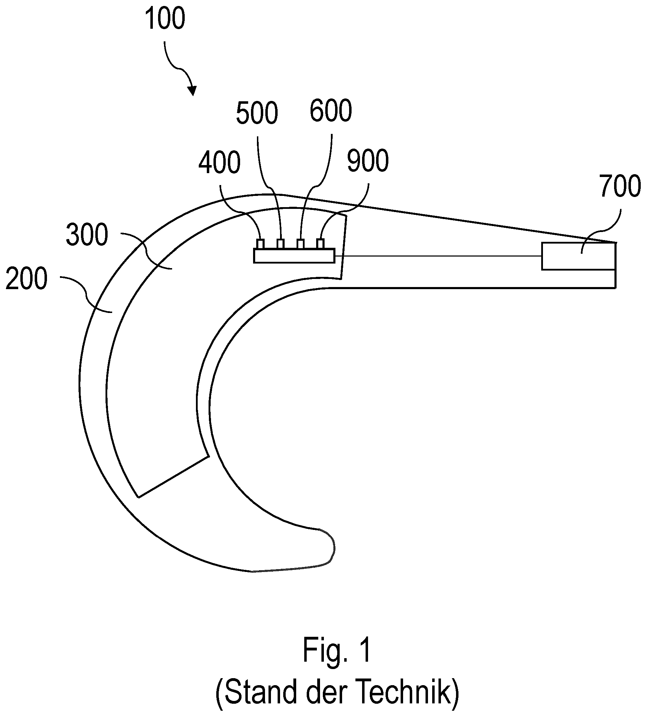

Fig. 1 zeigt einen im Stand der Technik bekannten Schlaghandschuh 100, der dazu ausgebildet ist, eine Schlagkraft zu bestimmen. Der bekannte Schlaghandschuh 100 umfasst hierfür einen Dämpfungskörper 200, einen fluidgefüllten Körper 300 sowie einen im fluidgefüllten Körper 300 angeordneten Drucksensor 400. Schlägt ein Athlet mit dem Schlaghandschuh 100 gegen ein Hindernis, wird eine Kraft auf den fluidgefüllten Körper 300 ausgeübt. Dadurch steigt der hydrostatische Druck im fluidgefüllten Körper 300, was mit dem Drucksensor 400 gemessen werden kann. Der Drucksensor 400 kommuniziert mit einer Recheneinheit 500, die aus den gemessenen Druckdaten eine Schlagkraft ermittelt. Der bekannte Schlaghandschuh 100 umfasst weiters einen Beschleunigungssensor 600, mittels welchem eine Schlagtrajektorie und somit weitere Informationen über einen zu vermessenden Schlag liefern kann. Zudem umfasst der bekannte Schlaghandschuh 100 einen Sendeempfänger 900, der die gemessenen oder berechneten Daten an eine externe Auswerteeinheit übermitteln kann, und eine Batterie 700, welche die genannten Komponenten mit elektrischer Energie versorgt. Es ist ersichtlich, dass alle genannten elektronischen Komponenten innerhalb des fluidgefüllten Körpers oder zumindest innerhalb des Schlaghandschuhes vorgesehen sind und daher nicht auswechselbar sind.

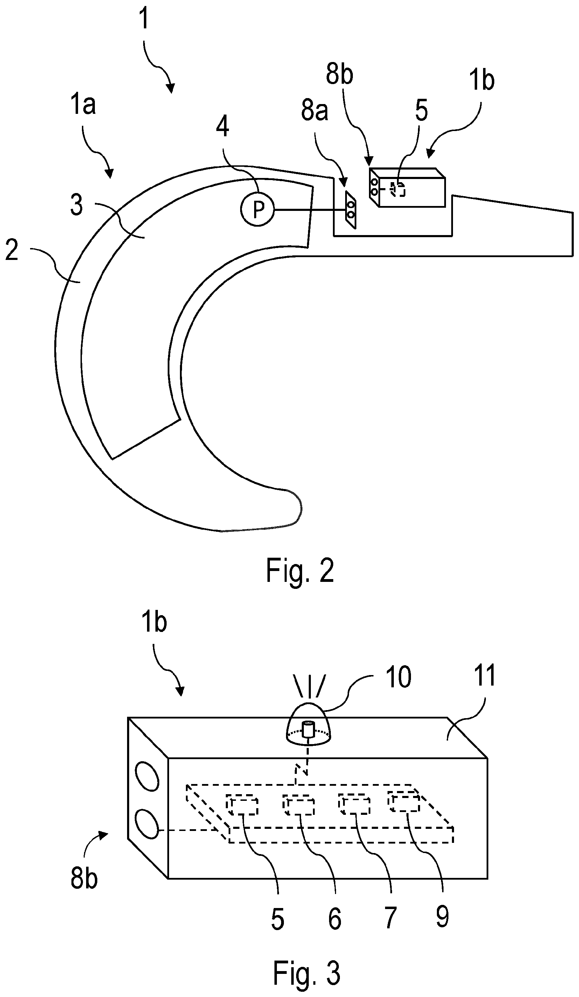

Fig. 2 zeigt ein erfindungsgemäßes modulares Schlagequipment 1, insbesondere einen Boxhandschuh, einen Karate-, Muay-Thai- oder einen Kickboxhandschuh, umfassend einen Schlagkörper 1a und ein Elektronikmodul 1b. Die Erfindung ist jedoch nicht auf Handschuhe beschränkt. Vielmehr kann eine Fachperson die Erfindung leicht auf andere Ausführungsformen übertragen, wo beispielsweise anstatt dem Schlag eines Armes ein Tritt eines Beins gemessen wird. Auch vorstellbar ist, dass die Erfindung als passives Sportinstrument, beispielsweise als Boxsack, ausgebildet ist. Im Folgenden bezieht sich die weitere Beschreibung auf die Ausführungsform als Schlaghandschuh, insbesondere um die Unterschiede gegenüber dem Stand der Technik zu verdeutlichen. Es versteht sich jedoch, dass alle hierin offenbarten Ausführungsformen für ein allgemeines Schlagequipment eingesetzt werden können.

Der erfindungsgemäße Schlaghandschuh 1 ist dazu ausgebildet, eine Schlagbeschleunigung, Schlaggeschwindigkeit, Schlagkraft und/oder Schlagtechnik zu ermitteln. Hierfür umfasst der erfindungsgemäße modulare Schlaghandschuh 1 einen Dämpfungskörper 2, einen fluidgefüllten Körper 3 (auch Pad oder Sensorpad genannt) sowie einen im fluidgefüllten Körper 3 angeordneten Drucksensor 4. Schlägt ein Athlet mit dem modularen Schlaghandschuh 1 gegen ein Hindernis, wird eine Kraft auf den fluidgefüllten Körper 3 ausgeübt. Dadurch steigt der hydrostatische Druck im fluidgefüllten Körper 3, was mit dem Drucksensor 4 gemessen werden kann. Der fluidgefüllte Körper 3 kann aus einem elastischen Material gefertigt sein, sodass er sich innerhalb gewisser Grenzwerte verformen kann. Beispielsweise kann der fluidgefüllte Körper 3 aus PVC, Neopren, Silikon oder thermoplastischen Elastomeren gefertigt sein. Der fluidgefüllte Körper 3 ist üblicherweise mit Luft gefüllt, könnte aber auch mit einem anderen Gas oder auch mit einer Flüssigkeit gefüllt sein.

Der genannte Dämpfungskörper 2 liegt zwischen einer Aufprallfläche des Schlaghandschuhs 1 und dem fluidgefüllten Körper 3 vor. Der Dämpfungskörper 2 setzt sich üblicherweise zusammen aus einer Außenhülle und einem Schaumstoffkörper, wobei die Außenhülle in den meisten Fällen eine Lederschicht oder eine Kunstlederschicht, d.h. eine Kunststofflage, ist. Der Dämpfungskörper 2 könnte jedoch auch anders ausgestaltet sein.

Ähnlich wie bei dem bekannten Schlaghandschuh 100 kommuniziert beim erfindungsgemäßen modularen Schlaghandschuh 1 der Drucksensor 4 mit einer Recheneinheit 5. Diese kann eine Schlagkraft auf Basis der vom Drucksensor 4 erhaltenen Druckmessdaten und einer bekannten Querschnittsfläche des fluidgefüllten Körpers 3 ermitteln. Im Gegensatz zum bekannten Schlaghandschuh 100 ist die Recheneinheit 5 bei der erfindungsgemäßen Lösung jedoch modular ausgebildet, d.h. austauschbar am Schlaghandschuh 1 vorgesehen. Hierfür wird das Elektronikmodul 1b zur Verfügung gestellt, welches zumindest die Recheneinheit 5 und den Beschleunigungssensor 6 umfasst und ferner eine Batterie 7 oder andere elektronische Komponenten umfassen kann (Figur 3). Das Elektronikmodul 1b ist derart ausgestaltet, dass es vom Schlagkörper 1a getrennt werden kann und daher auswechselbar an diesem vorliegt. Dies hat den Vorteil, dass alle jene elektronischen Komponenten, die im Elektronikmodul 1b vorliegen, nicht unlöslich im Schlaghandschuh 1 vorliegen und daher leicht eingebaut, repariert, ausgetauscht, aufgeladen etc. werden können.

Der Schlagkörper 1a und das Elektronikmodul 1b weisen jeweils eine Schnittstelle 8a, 8b auf, über die sie miteinander verbunden werden können. Konkret weist der Schlagkörper 1a eine erste Schnittstelle 8a auf und das Elektronikmodul 1b eine zweite Schnittstelle 8b.

Der Schlagkörper 1a und das Elektronikmodul 1b können somit in einen zusammengesetzten Zustand und in einen getrennten Zustand gebracht werden. Im zusammengesetzten Zustand sind die Schnittstellen 8a, 8b verbunden, und es kann zumindest eine Kommunikation zwischen dem Drucksensor 4 und der Recheneinheit 5 erfolgen. Im getrennten Zustand sind die Schnittstellen 8a, 8b voneinander getrennt und es kann keine Kommunikation zwischen dem Drucksensor 4 und der Recheneinheit 5 erfolgen.

Im einfachsten Fall sind die Schnittstellen 8a, 8b durch gegengleiche elektrische Kontakte ausgebildet, die sich im zusammengesetzten Zustand physisch berühren. Die elektrischen Kontakte können flächig ausgeführt sein oder auch als Steckverbindung, z.B. Klinkenverbindung, USB-Verbindung, Federkontaktstifte oder dergleichen. Im zusammengesetzten Zustand liegen die elektrischen Kontakte 8b des Elektronikmoduls 1b an jenen des Schlagkörpers 1a an, sodass sie sich gegenseitig berühren bzw. abdecken. Wenn das Elektronikmodul 1b keine weiteren elektrischen Kontakte umfasst, sind keine elektrischen Kontakte freiliegend, sobald der zusammengesetzte Zustand hergestellt wurde.

Es ist jedoch nicht zwingend erforderlich, dass die Schnittstellen 8a, 8b als elektrische Kontakte ausgebildet sind. Es ist denkbar, dass die Schnittstellen 8a, 8b eine drahtlose Kommunikation, etwa eine Nahfeldkommunikation (NFC), ermöglichen. In diesem Fall müssen sich die Schnittstellen 8a, 8b nicht unbedingt unmittelbar berühren, sondern lediglich physisch nahe aneinander positioniert werden, sodass eine drahtlose Kommunikation erfolgen kann. Hierbei kann beispielsweise verstanden werden, dass sich der Schlagkörper 1a und das Elektronikmodul 1b im zusammengesetzten Zustand befinden, wenn diese in einem Abstand angeordnet sind, der geringer als eine vorgegebene Schwellwertdistanz ist, und sich im getrennten Zustand befinden, wenn diese in einem Abstand angeordnet sind, der größer als die vorgegebene Schwellwertdistanz ist. Die Schwellwertdistanz ist hierbei jener Abstand, bis zu dem eine Kommunikation aufbaubar und/oder durchführbar ist.

Es sei noch erwähnt, dass, wenn die Schnittstellen durch drahtlose Kommunikationsmittel gebildet sind, es empfehlenswert ist, wenn der Schlagkörper neben dem Drucksensor auch einen Sendeempfänger und ein Datenverarbeitungselement umfasst. Die drahtlose Kommunikation könnte neben den Daten auch Energie übertragen, wie es etwa bei Nahfeldkommunikation (NFC) der Fall ist. Die drahtlose Kommunikation benötigt mehr Energie durch Erzeugen eines elektromagnetischen Feldes. Somit ist es bevorzugt, dass bei einer drahtlosen Schnittstelle diese nur während einer (potentiellen) Schlagbewegung Energie überträgt, sonst aber die meiste Zeit keine Kommunikation durchführt ist. Alternativ oder zusätzlich könnte zyklisch, beispielsweise alle x Sekunden getestet werden, ob eine Verbindung zum Drucksensor besteht.

Durch das Zusammenführen der Schnittstellen 8a, 8b wird der zusammengesetzte Zustand des Elektronikmoduls 1b und des Schlagkörpers 1a hergestellt, wobei der Drucksensor 4 des Schlagkörpers 1a mit der Recheneinheit 5 des Elektronikmoduls 1b verbunden wird. Im zusammengesetzten Zustand kann das Elektronikmodul 1b, genauer die Recheneinheit 5, somit Druckmessdaten erhalten, die vom Drucksensor 4 im fluidgefüllten Körper 3 des Schlagkörpers 1a gemessen wurden. Dadurch kann die Recheneinheit 5 zusammen mit einer Querschnittsfläche des fluidgefüllten Körpers 3, die in der Recheneinheit 5 hinterlegt sein kann, eine Schlagkraft bestimmen. Da das Elektronikmodul 1b auch mit unterschiedlichen Schlagkörpern 1a verbunden werden könnte, könnten das Elektronikmodul 1b und der Schlagkörper 1a über die Schnittstellen 8a, 8b auch eine Identifikation des Schlagkörpers 1a oder andere Daten wie z.B. die Querschnittsfläche des fluidgefüllten Körpers 3 über die Schnittstellen 8a, 8b austauschen.

Über die vom Beschleunigungssensor 6 gemessenen Beschleunigungsmessdaten kann auch eine Schlagbeschleunigung, Schlaggeschwindigkeit, Schlagkraft (über eine geschätzte effektive Masse, z.B. zur Verifizierung der mittels der Druckmessdaten bestimmten Schlagkraft) oder Schlagtechnik bestimmt werden.

In den oben erläuterten Ausführungsformen liegt der Beschleunigungssensor 6 im Elektronikmodul 1b vor.

Fig. 3 zeigt eine bevorzugte Ausführungsform des erfindungsgemäßen Elektronikmoduls 1b. Das Elektronikmodul 1b umfasst im dargestellten Beispiel die Recheneinheit 5, den Beschleunigungssensor 6 und die Batterie 7.

Die Recheneinheit 5 kann als einfacher integrierter Schaltkreis ausgeführt oder auch nur als simple Logikschaltung implementiert sein. Alternativ könnte es sich auch um einen Computer mit darauf gespeichertem Computerprogramm handeln.

Der Beschleunigungssensor 6 ist in der Regel dazu ausgebildet, Beschleunigungsmesswerte in drei orthogonal zueinanderstehenden Raumrichtungen x, y, z aufzuzeichnen. Der Beschleunigungssensor 6 kann insbesondere Teil einer IMU (Intertial Measurement Unit) sein, die auch einen Drehratensensor umfassen kann.

Die Batterie 7 (unabhängig ob diese im Elektronikmodul 1b oder außerhalb des Elektronikmoduls 1b vorliegt) bietet eine Stromversorgung für die Recheneinheit 5 und gegebenenfalls auch den Beschleunigungssensor 6. Wenn der Drucksensor 4 im Einsatz Strom benötigt, kann die Batterie 7 dem Drucksensor 4 den Strom im zusammengesetzten Zustand auch über die Schnittstellen 8a, 8b zur Verfügung stellen.

Im Beispiel von Figur 2 können Beschleunigungen durch den Beschleunigungssensor 6 gemessen und in der Recheneinheit 5 verarbeitet werden. In der gezeigten Ausführungsform sind die Recheneinheit 5, der Beschleunigungssensor 6 und die Batterie 7 an einer gemeinsamen Platine montiert. Der Aufbau des Elektronikmoduls 1b ist jedoch nicht auf die gezeigte Ausführungsform beschränkt, sodass beispielsweise die genannte Platine auch entfallen könnte oder es mehr als eine Platine geben könnte. Ist die Batterie 7 wie dargestellt permanent im Elektronikmodul 1b vorgesehen, kann diese als wiederaufladbarer Akkumulator ausgeführt sein. Alternativ könnte die Batterie 7 auch auswechselbar im Elektronikmodul 1b vorgesehen sein, z.B. als eine oder mehrere Knopfzellen, AAA-Batterien oder dergleichen.

Das Elektronikmodul 1b umfasst zudem die genannte Schnittstelle 8b, über die eine Verbindung des Elektronikmoduls 1b mit dem Schlagkörper 1a hergestellt werden kann (siehe Fig. 2). In der dargestellten Variante ist die Schnittstelle 8b des Elektronikmoduls 1b durch zwei flächige elektrische Kontakte ausgeführt.

Weiters kann das Elektronikmodul 1b einen Sendeempfänger 9 aufweisen, welcher dazu konfiguriert ist, von der Recheneinheit 5 ermittelte Daten an eine externe (in Fig. 3 nicht gezeigte) Datenverarbeitungseinheit zu senden. In einem praktischen Beispiel kann die Datenverarbeitungseinheit ein Mobiltelefon sein, sodass die ermittelte Schlageigenschaft über eine Applikation des Mobiltelefons ausgebbar ist, z.B. in einem Live-Modus oder in einem Analysemodus nach der Benutzung des Schlaghandschuhes 1.

Die Kommunikation des Sendeempfängers 9 mit der externen Datenverarbeitungseinheit kann beispielsweise drahtlos, etwa über eine Funkverbindung, oder kabelgebunden geschehen, etwa indem die kabelgebundene Kommunikation über die genannte Schnittstelle 8b des Elektronikmoduls 1b oder eine andere Schnittstelle des Elektronikmoduls 1b erfolgt. Es kann auch vorgesehen sein, dass der Sendeempfänger 9 Daten von der externen Datenverarbeitungseinheit empfängt. Das kann beispielsweise ein Wert für eine effektive Masse sein, mithilfe derer aus einer Schlagbeschleunigung eine Schlagkraft berechnet werden kann. Alternativ dazu oder zusätzlich kann es vorgesehen sein, dass der Sendeempfänger 9 einen Befehl erhält, wodurch ein physischer Kopplungs- oder Entkopplungsvorgang zu bzw. von dem Schlagkörper 1a ausgelöst wird.

In vielen Fällen wird das Elektronikmodul 1b auch eine Speichereinheit aufweisen, in welcher gesammelte Daten, insbesondere die Schlageigenschaft und/oder Druckmessdaten und/oder Beschleunigungsmessdaten hinterlegt werden können. Der genannte Sendeempfänger 9 kann in dieser Variante Daten von der Speichereinheit an die externe Datenverarbeitungseinheit senden oder von dieser empfangen.

Die Speichereinheit kann beispielsweise als FIFO (First In First Out) ausgestaltet sein, wodurch leicht ermöglicht wird, dass immer die jeweils x letzten Sekunden oder Minuten aufgezeichnet werden. Alternativ oder zusätzlich kann die Speichereinheit dazu ausgestaltet sein, dass immer die x letzten jeweils abgeschlossenen Bewegungsaufzeichnungen von Schlägen aufgezeichnet werden.

Das Elektronikmodul 1b kann ferner wie dargestellt einen Signalindikator 10 aufweisen. Bevorzugt ist der Signalindikator 10 als LED-Anzeige ausgeführt, es ist jedoch auch denkbar, dass es sich bei dem Signalindikator 10 um einen akustischen, haptischen und/oder olfaktorischen Signalindikator 10 handelt, sodass beispielsweise der Benutzer durch einen Ton, eine Melodie, eine Vibration, ein vorbestimmtes Vibrationsmuster oder einen Duft auf einen gewissen Betriebszustand hingewiesen wird. Auch eine Kombination der genannten Signalindikatoren 10 ist denkbar.

Vorzugsweise kann ein Kopplungs- oder Entkopplungsvorgang des Elektronikmoduls 1b zu bzw. von einem Schlagkörper 1a einem Benutzer über den Signalindikator 10 angezeigt werden. Optional kann ein aufgetretener Fehler beim Kopplungsvorgang über den Signalindikator 10 mitgeteilt werden. Alternativ oder zusätzlich kann das Bestehen oder das Nichtbestehen einer Verbindung über den Signalindikator 10 einem Benutzer mitgeteilt werden. In einem praktischen Beispiel kann eine LED-Anzeige ein Licht anzeigen, insbesondere ein grünes Licht, wenn sich der Schlagkörper 1a und das Elektronikmodul 1b in einem zusammengesetzten Zustand befinden. Bei einem Verbindungsaufbau könnte die LED-Anzeige blinken.

Es ist auch möglich, dass der Signalindikator 10 dem Benutzer einen Ladezustand der Batterie 7 und/oder andere Betriebszustände (z.B. Funktionstüchtigkeit des Beschleunigungssensors 6, freier Speicherplatz der Speichereinheit etc.) mitteilen kann. Alternativ oder zusätzlich kann der Signalindikator 10 mitteilen, dass die Batterie gerade aufgeladen wird. Alternativ oder zusätzlich kann der Signalindikator 10 auf einer externen Datenverarbeitungseinheit, beispielsweise einem Smartphone, realisiert sein.

Das Elektronikmodul 1b weist ferner ein Gehäuse 11 auf, welches zumindest die Recheneinheit 5, den Beschleunigungssensor 6 und die Batterie 7 umschließt. Dieses Gehäuse 11 kann beispielsweise ein Kunststoffgehäuse oder ein Metallgehäuse sein und ist vorzugsweise wasserdicht ausgebildet, sodass die vom Gehäuse 11 umschlossenen elektronischen Komponenten zumindest vor Spritzwasser geschützt sind. In der gezeigten Ausführungsform ist das Gehäuse 11 als Quader ausgebildet, wobei die Form des Gehäuses ist jedoch nicht auf Quader beschränkt ist. In jener Variante, in der die Schnittstellen 8a, 8b als elektrische Kontakte ausgeführt sind, liegt die Schnittstelle 8b des Elektronikmoduls 1b an einer Außenseite des Gehäuses 11 vor. Bevorzugt sind dies die einzigen an der Außenseite des Gehäuses 11 vorliegenden Kontakte, da hierbei keine nach außen freiliegenden elektrischen Kontakte vorliegen werden, wenn die Schnittstelle 8b des Elektronikmoduls 1b an der Schnittstelle 8a des Schlagkörpers 1a anliegt.

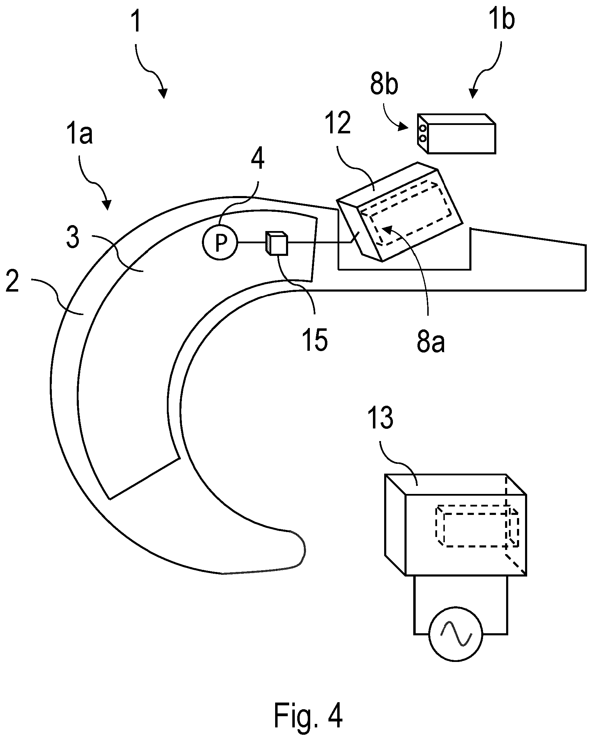

Um das Elektronikmodul 1b physisch mit dem Schlagkörper 1a zu verbinden, und insbesondere um eine stabile Verbindung der Schnittstellen 8a, 8b herzustellen, können das Elektronikmodul 1b und der Schlagkörper 1a beispielsweise durch einen Klemm-, Magnet-, Saugnapf- oder Klettverschluss zusammengehalten werden. Diese Varianten können auch dann zum Einsatz kommen, wenn die physischen Verbindungsflächen im Wesentlichen eben ausgeführt sind, wie in Fig. 1 dargestellt ist. Im Folgenden wird jedoch anhand von Fig. 4 eine bevorzugte Variante dargestellt, bei welcher das Elektronikmodul 1b in eine Aufnahmeeinheit 12 einführbar ist, wodurch eine besonders stabile physische Verbindung erzielt werden kann.

In Fig. 4 ist eine weitere bevorzugte Ausführungsform des modularen Schlaghandschuhs 1 zusammen mit einer optionalen Ladeeinheit 13 für das Elektronikmodul gezeigt. Die in Fig. 4 gezeigte Ausführungsform des modularen Schlaghandschuhs 1 unterscheidet sich von der in Fig. 2 gezeigten Ausführungsform des modularen Schlaghandschuhs 1 dadurch, dass der Schlagkörper 1a eine Aufnahmeeinheit 12 umfasst, in die das Elektronikmodul 1b zumindest teilweise einführbar ist. In der gezeigten Ausführungsform ist die Aufnahmeeinheit 12 als Quader ausgebildet, der eine Ausnehmung aufweist, in die das Elektronikmodul 1b eingeführt werden kann. Die Form der Aufnahmeeinheit 12 ist jedoch nicht auf Quader beschränkt.

Weiters ist aus Fig. 4 ersichtlich, dass der Schlagkörper 1a eine zweite Recheneinheit 15 aufweist. Die zweite Recheneinheit 15 empfängt die vom Drucksensor 4 gemessenen Druckmesswerte und ist mit der Schnittstelle 8a des Schlagkörpers 1a verbunden. Somit ist die mit dem Drucksensor 4 verbundene, im Schlagkörper 1a vorliegende zweite Recheneinheit 15 mit der zumindest einen elektronischen Komponente des Elektronikmoduls 1b verbindbar. Der Schlagkörper 1a kann somit (unabhängig vom Elektronikmodul 1b) mittels der zweiten Recheneinheit 15 selbstständig eine Schlageigenschaft ermitteln oder Daten an eine externe Recheneinheit senden.

An dieser Stelle sei angemerkt, dass die zweite Recheneinheit 15 unabhängig von der Aufnahmeeinheit 12 ist. Es sind Ausführungsformen denkbar, bei denen eine in Fig. 4 gezeigte Aufnahmeeinheit 12 vorliegt, jedoch keine zweite Recheneinheit 15. Ebenso ist es denkbar, dass eine andere Ausführungsform eine in Fig. 4 gezeigte zweite Recheneinheit 15 aufweist, jedoch keine Aufnahmeeinheit 12. In anderen Worten kann die zweite Recheneinheit 15 auch in der in Fig. 2 gezeigten Ausführungsform vorgesehen sein, ohne dass diese Ausführungsform eine Aufnahmeeinheit 12 aufweist. Ebenso kann die Aufnahmeeinheit 12 in der in Fig. 2 gezeigten Ausführungsform vorgesehen sein, ohne dass diese Ausführungsform eine zweite Recheneinheit 15 aufweist.

Durch das Einführen des Elektronikmoduls 1b in die Aufnahmeeinheit 12 erfolgt das physische Zusammenbringen der Schnittstellen 8a, 8b, sodass der zusammengesetzte Zustand hergestellt wird. Falls die Schnittstellen 8a, 8b als elektrische Kontakte ausgebildet sind, weist die Aufnahmeeinheit 12 in ihrem Inneren ebenfalls elektrische Kontakte auf. Falls die Schnittstellen 8a, 8b eine drahtlose Kommunikation ermöglichen, weist die Schnittstelle 8a der Aufnahmeeinheit 12 entsprechende Kommunikationsmittel auf. Bevorzugt umfasst die Aufnahmeeinheit 12 Dichtungselemente, welche die Schnittstellen 8a, 8b im zusammengesetzten Zustand wasserdicht im Innenraum der Aufnahmeeinheit 12 einschließen. Falls die Schnittstellen 8a, 8b als elektrische Kontakte ausgebildet sind, sind sie dadurch von Flüssigkeiten, beispielsweise Regenwasser oder Schweiß, geschützt.

Das Elektronikmodul 1b kann über verschiedene Arten in der Aufnahmeeinheit 12 befestigt werden. Beispielsweise kann dies über einen Klemm-, Magnet-, Saugnapf- oder Klettverschluss geschehen. Alternativ oder zusätzlich kann die Aufnahmeeinheit 12 einen (in Fig. 4 nicht gezeigten) Deckel oder eine Klappe aufweisen, die dazu ausgebildet sind, das Elektronikmodul 1b vollständig in der Aufnahmeeinheit 12 einzuschließen und ein Austreten des Elektronikmoduls 1b aus der Aufnahmeeinheit 12 zu verhindern.

Für die Beschreibung der restlichen in Fig. 4 gezeigten Elemente des modularen Schlaghandschuhs 1 wird auf die Beschreibung der Figuren 2 und 3 verwiesen.

Bevorzugt wird durch die Erfindung nicht nur ein modularer Schlaghandschuh 1, sondern auch ein System aus einem modularen Schlaghandschuh 1 und einer Ladeeinheit 13 bereitgestellt.

Die Ladeeinheit 13 ist dazu ausgebildet, dass sie mit einem Elektronikmodul 1b im getrennten Zustand verbunden werden kann. In anderen Worten kann man in der gezeigten Ausführungsform das Elektronikmodul 1b entweder mit dem Schlagkörper 1a verbinden (wodurch der zusammengesetzte Zustand hergestellt wird) oder mit der Ladeeinheit 13 verbinden.

Die Ladeeinheit 13 ist ferner dazu konfiguriert, die Batterie 7 des Elektronikmoduls 1b über die Schnittstelle 8b des Elektronikmoduls 1b aufzuladen und/oder Daten mit dem Elektronikmodul 1b auszutauschen. Vorzugsweise ermöglicht die Schnittstelle 8b des Elektronikmoduls 1b also sowohl eine Kommunikation mit dem Drucksensor 4 eines Schlagkörpers 1a als auch eine Stromverbindung zu einer Ladeeinheit 13. Die Ladeeinheit 13 könnte die Batterie 7 des Elektronikmoduls 1b aber auch drahtlos, beispielsweise induktiv, aufladen.

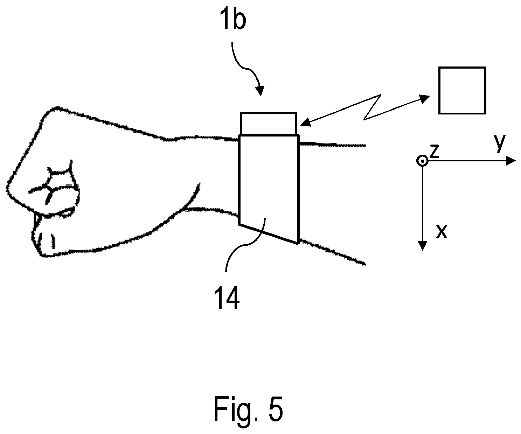

Fig. 5 zeigt eine Ausführungsform, bei der das Elektronikmodul 1b als selbstständig einsatzfähiges Modul eingesetzt werden kann, welches auch eine Schlageigenschaft ermittelt, wenn kein Drucksensor 4 mit diesem verbunden ist. Wenn dieses Elektronikmodul 1b im getrennten Zustand an einem Körperteil eines Athleten montiert wird, kann der Beschleunigungssensor 6 die Beschleunigung des entsprechenden Körperteils messen. Zusammen mit einer geschätzten effektiven Masse kann daraus die Schlagkraft berechnet werden. Es versteht sich jedoch, dass diese Ermittlung wesentlich ungenauer ist als bei einer Messung mit angeschlossenem Drucksensor 4.

Wird das Elektronikmodul 1b als selbstständig einsatzfähiges Modul eingesetzt, umfasst dieses jedenfalls einen Beschleunigungssensor 6, der mit der Recheneinheit 5 kommuniziert, sodass basierend auf den gemessenen Beschleunigungsdaten auch eine Schlaggeschwindigkeit, Schlagkraft oder Schlagtechnik durch die Recheneinheit 5 ermittelt werden kann.

Wie in Fig. 5 dargestellt ist, kann das Elektronikmodul 1b beispielsweise mittels einer Bandage 14 oder einem Handschuh am Unterarm oder einem anderen Körperteil eines Athleten montiert werden, sodass etwa beim Boxen oder einer ähnlichen Sportart eine Schlagbeschleunigung gemessen werden kann. In dieser Ausführungsform wird üblicherweise die Schnittstelle 8b des Elektronikmoduls 1b zum Drucksensor 4 unbenutzt freiliegen, wobei diese aber auch in der Bandage 14 aufgenommen und damit von dieser abgedeckt werden kann. Sind die Kontakte in dieser Ausführungsform verdeckt, können ebenso Schäden wie durch andere Einflüsse, etwa ESD, vermieden werden. Das Elektronikmodul 1b könnte auch in eine Tasche eines Schützers wie eines Fußschützers eingeführt werden, sodass keine Bandage 14 benötigt wird.

Mit dieser Ausführungsform kann gegebenenfalls die Batterielaufzeit des Elektronikmoduls 1b erhöht werden, insbesondere wenn der Drucksensor 4 im Einsatz Strom benötigt.

## Patentansprüche

1. Modulares Schlagequipment (1), bevorzugt modularer Schlaghandschuh, zur Bestimmung einer Schlageigenschaft, insbesondere einer Schlagbeschleunigung, Schlaggeschwindigkeit, Schlagkraft oder Schlagtechnik, umfassend einen Schlagkörper (1a) und ein Elektronikmodul (1b), wobei der Schlagkörper (1a) einen Dämpfungskörper (2) und einen Drucksensor (4) umfasst, wobei das Elektronikmodul (1b) zumindest eine elektronische Komponente, bevorzugt eine erste Recheneinheit (5) zur Bestimmung der Schlageigenschaft, umfasst,
wobei das Elektronikmodul (1b) und der Schlagkörper (1a) jeweils eine Schnittstelle (8a, 8b) aufweisen, über welche der Drucksensor (4) oder eine mit dem Drucksensor (4) verbundene, im Schlagkörper (1a) vorliegende zweite Recheneinheit (15) mit der zumindest einen elektronischen Komponente des Elektronikmoduls (1b) verbindbar ist,
wobei das Elektronikmodul (1b) und der Schlagkörper (1a) physisch an den genannten Schnittstellen (8a, 8b) voneinander trennbar und zusammensetzbar sind, sodass das Elektronikmodul (1b) und der Schlagkörper (1a) in einen zusammengesetzten Zustand und in einen getrennten Zustand bringbar sind,
wobei das modulare Schlagequipment (1) in einem zusammengesetzten Zustand dazu ausgebildet ist, Daten und/oder Energie zwischen der zumindest einen elektronischen Komponente und dem Drucksensor (4) und/oder der zweiten Recheneinheit (15) über die genannten Schnittstellen (8a, 8b) auszutauschen,
**dadurch gekennzeichnet, dass**
das Elektronikmodul (1b) die erste Recheneinheit (5) und einen Beschleunigungssensor (6) als elektronische Komponente umfasst, wobei die erste Recheneinheit (5) dazu ausgebildet ist, in einem getrennten Zustand eine Schlageigenschaft über vom Beschleunigungssensor (6) erhaltene Beschleunigungsmessdaten zu ermitteln und/oder die Beschleunigungsmessdaten zur Ermittlung einer Schlageigenschaft an eine externe Recheneinheit zu senden.

2. Modulares Schlagequipment nach Anspruch 1, wobei der Schlagkörper (1a) einen fluidgefüllten Körper (3) umfasst, in welchem der Drucksensor angeordnet ist, um einen hydrostatischen Druck im fluidgefüllten Körper (3) zu messen.

3. Modulares Schlagequipment nach Anspruch 1 oder 2, wobei die erste Recheneinheit (5) dazu ausgebildet ist, eine Schlageigenschaft über vom Drucksensor (4) erhaltenen Druckmessdaten zu ermitteln und/oder die vom Drucksensor (4) erhaltenen Druckmessdaten zur Ermittlung einer Schlageigenschaft an eine externe Recheneinheit zu senden, wenn sich das Elektronikmodul (1b) und der Schlagkörper (1a) im zusammengesetzten Zustand befinden.

4. Modulares Schlagequipment nach einem der Ansprüche 1 bis 3, wobei der Schlagkörper (1a) die zweite Recheneinheit (15) zur Bestimmung der Schlageigenschaft auf Basis der vom Drucksensor (4) gelieferten Messdaten und/oder einen Sendeempfänger umfasst.

5. Modulares Schlagequipment nach einem der Ansprüche 1 bis 4, wobei das Elektronikmodul (1b) einen Signalindikator (10), bevorzugt eine LED-Anzeige und/oder ein LCD-Display, zum Mitteilen eines Betriebszustandes des Elektronikmoduls (1b) umfasst.

6. Modulares Schlagequipment nach Anspruch 5, wobei der Signalindikator (10) einem Benutzer einen Verbindungszustand zum Drucksensor (4) und/oder zur zweiten Recheneinheit (15), vorzugsweise das Herstellen einer Verbindung, das Bestehen einer Verbindung und/oder das Nichtbestehen einer Verbindung, mitteilen kann.

7. Modulares Schlagequipment nach einem der Ansprüche 1 bis 6, wobei die Schnittstellen (8a, 8b) durch drahtlose Kommunikationsmittel gebildet sind.

8. Modulares Schlagequipment nach einem der Ansprüche 1 bis 7, wobei das Elektronikmodul (1b) eine Batterie (7) als elektronische Komponente umfasst, wobei die Batterie (7) im getrennten Zustand über die genannte Schnittstelle (8b) des Elektronikmoduls (1b) aufladbar ist.

9. Modulares Schlagequipment nach einem der Ansprüche 1 bis 8, wobei das Elektronikmodul (1b) weiters ein Gehäuse (11) umfasst, welches alle elektronischen Komponenten des Elektronikmoduls (1b), bevorzugt wasserdicht und/oder staubdicht, umschließt.

10. Modulares Schlagequipment nach einem der Ansprüche 1 bis 9, wobei alle elektrischen Kontakte, insbesondere Ladekontakte, des Elektronikmoduls (1b) im zusammengesetzten Zustand an der Schnittstelle (8b) des Elektronikmoduls (1b) abgedeckt sind.

11. Modulares Schlagequipment nach einem der Ansprüche 1 bis 10, wobei der Schlagkörper (1a) weiters eine Aufnahmeeinheit (12) umfasst, in welche das Elektronikmodul (1b) einführbar ist, wobei im zusammengesetzten Zustand das Elektronikmodul (1b) in der Aufnahmeeinheit (12) zumindest teilweise eingeführt ist.

12. Modulares Schlagequipment nach Anspruch 11, wobei die Aufnahmeeinheit (12) Dichtungselemente umfasst, welche die Schnittstellen (8a, 8b) im zusammengesetzten Zustand wasserdicht und/oder staubdicht im Innenraum der Aufnahmeeinheit (12) einschließen.

13. System aus einem modularen Schlagequipment nach einem der Ansprüche 1 bis 12 und einer Ladeeinheit (13), wobei das Elektronikmodul (1b) im getrennten Zustand mit der Ladeeinheit (13) verbindbar ist, wobei die Ladeeinheit (13) dazu konfiguriert ist, eine Batterie (7) des Elektronikmoduls (1b) über die Schnittstelle (8b) des Elektronikmoduls (1b) aufzuladen.

## Claims

1. Modular striking equipment (1), preferably a modular striking glove, for determining a striking characteristic, in particular striking acceleration, striking velocity, striking force or striking technique, comprising a striking body (1a) and an electronic module (1b), wherein the striking body (1a) comprises a damping body (2) and a pressure sensor (4), and the electronic module (1b) comprises at least one electronic component, preferably a first processing unit (5), for determining the striking characteristic,
wherein the electronic module (1b) and the striking body (1a) each have an interface (8a, 8b) via which the pressure sensor (4) or a second processing unit (15) connected to the pressure sensor (4) and located within the striking body (1a) can be connected to the at least one electronic component of the electronic module (1b),
wherein the electronic module (1b) and the striking body (1a) can be physically separated from and reassembled with one another at the said interfaces (8a, 8b), such that the electronic module (1b) and the striking body (1a) can be brought into an assembled state and into a separated state, wherein the modular striking equipment (1), when in an assembled state, is designed to exchange data and/or energy between the at least one electronic component and the pressure sensor (4) and/or the second processing unit (15) via the said interfaces (8a, 8b),
**characterised in that**
the electronics module (1b) comprises the first processing unit (5) and an acceleration sensor (6) as electronic components, wherein the first processing unit (5) is configured, in a separated state, to determine a striking characteristic based on acceleration measurement data received from the acceleration sensor (6) and/or to transmit the acceleration measurement data to an external processing unit for the purpose of determining a striking characteristic.

2. Modular striking equipment according to claim 1, wherein the striking body (1a) comprises a fluid-filled body (3) in which the pressure sensor is arranged to measure hydrostatic pressure within the fluid-filled body (3).

3. Modular striking equipment according to claim 1 or 2, wherein the first processing unit (5) is configured to determine a striking characteristic based on pressure measurement data obtained from the pressure sensor (4) and/or to transmit the pressure measurement data obtained from the pressure sensor (4) to an external processing unit for the purpose of determining a striking characteristic, when the electronic module (1b) and the striking body (1a) are in an assembled state.

4. Modular striking equipment according to any one of claims 1 to 3, wherein the striking body (1a) comprises the second processing unit (15) for determining the striking characteristic on the basis of the measurement data supplied by the pressure sensor (4) and/or a transceiver.

5. Modular striking equipment according to any one of claims 1 to 4, wherein the electronic module (1b) comprises a signal indicator (10), preferably an LED indicator and/or an LCD display, for indicating an operating state of the electronic module (1b).

6. Modular striking equipment according to claim 5, wherein the signal indicator (10) is capable of indicating to a user a connection status with the pressure sensor (4) and/or the second processing unit (15), preferably the establishment of a connection, the existence of a connection and/or the absence of a connection.

7. Modular striking equipment according to any one of claims 1 to 6, wherein the interfaces (8a, 8b) are formed by wireless communication means.

8. Modular striking equipment according to any one of claims 1 to 7, wherein the electronic module (1b) comprises a battery (7) as an electronic component, wherein the battery (7) can be charged whilst disconnected via the said interface (8b) of the electronic module (1b).

9. Modular striking equipment according to any one of claims 1 to 8, wherein the electronic module (1b) further comprises a housing (11) which encloses all the electronic components of the electronic module (1b), preferably in a watertight and/or dust-tight manner.

10. Modular striking equipment according to any one of claims 1 to 9, wherein all electrical contacts, in particular charging contacts, of the electronic module (1b) are covered at the interface (8b) of the electronic module (1b) when assembled.

11. Modular striking equipment according to any one of claims 1 to 10, wherein the striking body (1a) further comprises a housing unit (12) into which the electronic module (1b) can be inserted, wherein, in the assembled state, the electronic module (1b) is at least partially inserted into the housing unit (12).

12. Modular striking equipment according to claim 11, wherein the housing unit (12) comprises sealing elements which, in the assembled state, enclose the interfaces (8a, 8b) within the interior of the housing unit (12) in a watertight and/or dust-tight manner.

13. A system comprising modular striking equipment according to any one of claims 1 to 12 and a charging unit (13), wherein the electronic module (1b) can be connected to the charging unit (13) when separated, and wherein the charging unit (13) is configured to charge a battery (7) of the electronic module (1b) via the interface (8b) of the electronic module (1b).

## Revendications

1. Équipement de frappe modulaire (1), de préférence un gant de frappe modulaire, pour la détermination d'une caractéristique de frappe, notamment d'une acceleration de frappe, d'une vitesse de frappe, d'une force de frappe ou d'une technique de frappe, comprenant un corps de frappe (1a) et un module électronique (1b), le corps de frappe (1a) comprenant un corps d'amortissement (2) et un capteur de pression (4), le module électronique (1b) comprenant au moins un composant électronique, de préférence une première unité de calcul (5) pour la détermination de la caractéristique de frappe,
le module électronique (1b) et le corps de frappe (1a) présentant chacun une interface (8a, 8b) par laquelle le capteur de pression (4) ou une seconde unité de calcul (15) reliée au capteur de pression (4) et située dans le corps de frappe (1a) peut être relié à au moins un composant électronique du module électronique (1b),
le module électronique (1b) et le corps de frappe (1a) étant physiquement séparables l'un de l'autre et composables au niveau des interfaces susmentionnées (8a, 8b), de sorte que le module électronique (1b) et le corps de frappe (1a) peuvent être amenés dans un état assemblé et dans un état séparé,
l'équipement de frappe modulaire (1), à l'état assemblé, étant conçu pour échanger des données et/ou de l'énergie entre au moins un composant électronique et le capteur de pression (4) et/ou la seconde unité de calcul (15) via les interfaces susmentionnées (8a, 8b),
**caractérisé en ce que** le module électronique (1b) comprend la première unité de calcul (5) et un capteur d'accélération (6) en tant que composants électroniques, la première unité de calcul (5) étant conçue pour déterminer une caractéristique de frappe à l'état séparé par l'intermédiaire de données de mesure d'accélération reçues du capteur d'accélération (6) et/ou pour transmettre les données de mesure d'accélération à une unité de calcul externe afin de déterminer une caractéristique de frappe.

2. Équipement de frappe modulaire selon la revendication 1, dans lequel le corps de frappe (1a) comprend un corps rempli de fluide (3) dans lequel le capteur de pression est disposé pour mesurer une pression hydrostatique dans le corps rempli de fluide (3).

3. Équipement de frappe modulaire selon la revendication 1 ou 2, dans lequel la première unité de calcul (5) est conçue pour déterminer une caractéristique de frappe par l'intermédiaire de données de mesure de pression reçues du capteur de pression (4) et/ou pour transmettre les données de mesure de pression reçues du capteur de pression (4) à une unité de calcul externe pour déterminer une caractéristique de frappe lorsque le module électronique (1b) et le corps de frappe (1a) sont à l'état assemblé.

4. Équipement de frappe modulaire selon l'une quelconque des revendications 1 à 3, dans lequel le corps de frappe (1a) comprend la seconde unité de calcul (15) pour la détermination de la caractéristique de frappe à partir des données de mesure fournies par le capteur de pression (4) et/ou un émetteur-récepteur.

5. Équipement de frappe modulaire selon l'une quelconque des revendications 1 à 4, dans lequel le module électronique (1b) comprend un indicateur de signal (10), de préférence un affichage LED et/ou un écran LCD, permettant de communiquer un état de fonctionnement du module électronique (1b).

6. Équipement de frappe modulaire selon la revendication 5, dans lequel l'indicateur de signal (10) peut informer l'utilisateur d'un état de connexion au capteur de pression (4) et/ou à la seconde unité de calcul (15), de preference de l'établissement d'une connexion, de l'existence d'une connexion et/ou de l'absence d'une connexion.

7. Équipement de frappe modulaire selon l'une quelconque des revendications 1 à 6, dans lequel les interfaces (8a, 8b) sont formées par des moyens de communication sans fil.

8. Équipement de frappe modulaire selon l'une quelconque des revendications 1 à 7, dans lequel le module électronique (1b) comprend une batterie (7) en tant que composant électronique, la batterie (7) à l'état séparé étant rechargeable via l'interface susmentionnée (8b) du module électronique (1b).

9. Équipement de frappe modulaire selon l'une quelconque des revendications 1 à 8, dans lequel le module électronique (1b) comprend en outre un boîtier (11) qui renferme tous les composants électroniques du module électronique (1b), de préférence de manière étanche à l'eau et/ou de manière étanche à la poussière.

10. Équipement de frappe modulaire selon l'une quelconque des revendications 1 à 9, dans lequel tous les contacts électriques, notamment les contacts de charge, du module électronique (1b) à l'état assemblé sont protégés au niveau de l'interface (8b) du module électronique (1b).

11. Équipement de frappe modulaire selon l'une quelconque des revendications 1 à 10, dans lequel le corps de frappe (1a) comprend en outre une unité de logement (12) dans laquelle le module électronique (1b) peut être inséré, le module électronique (1b) étant, à l'état assemblé, au moins partiellement inséré dans l'unité de logement (12).

12. Équipement de frappe modulaire selon la revendication 11, dans lequel l'unité de logement (12) comprend des éléments d'étanchéité qui, à l'état assemblé, enferment les interfaces (8a, 8b) à l'intérieur de la l'unité de logement (12) de manière étanche à l'eau et/ou de manière étanche à la poussière.

13. Système composé d'un équipement de frappe modulaire selon l'une quelconque des revendications 1 à 12 et d'une unité de chargement (13), dans lequel le module électronique (1b) peut être relié à l'unité de chargement (13) à l'état séparé, l'unité de chargement (13) étant conçue pour charger une batterie (7) du module électronique (1b) via l'interface (8b) du module électronique (1b).
